# EUROPEAN PATENT APPLICATION

(11) **EP 3 138 905 A1**
(43) Date of publication of application: **08.03.2017**
(21) Application number: 15183968.5
(22) Date of filing: 04.09.2015
(51) Int. Cl.: C12N 5/0783

(54) **METHOD FOR NATURAL KILLER CELL EXPANSION**

(71) Applicant: Miltenyi Biotec GmbH, 51429 Bergisch Gladbach (DE)
(72) Inventor: GRANZIN, Markus, 41061 Mönchengladbach (DE); HUPPERT, Volker, 51515 Kürten (DE)
(74) Representative: Biervert, Christian

(57) **Abstract**

The present invention provides a method for in-vitro culturing and expanding natural killer (NK) cells in a cell culture medium comprising a population of NK cells, the method comprising a) adding an effective concentration of interleukin-21 (IL-21) at the beginning of the culturing process to said medium, b) adding repeatedly an effective concentration of interleukin-2 (IL-2) and/or interleukin-15 (IL-15) to said medium, and c) adding repeatedly feeder cells or membrane particles thereof to said medium, wherein said feeder cells are B cell derived which are EBV immortalized; and wherein said expansion of NK cells in said cell culture medium is maintained for at least 3 weeks.

## Description

### Field of the invention

The present invention relates to efficient multiplication of Natural Killer cell ("NK cell") numbers in ex-vivo culture and, more particularly, but not exclusively by treating the cells with feeder cells and cytokines.

### Background of the invention

Natural Killer cells (hereinafter also abbreviated as "NK cells") are a unique population of lymphocytes that is able to detect and destroy virus infected cells and malignantly degenerated cells, also known as tumor cells. Additionally NK cells produce and secrete cytokines upon contact with tumor cells. These functional feature makes NK cells an attractive drug for treatment of cancer. Clinical trials have emphasized the potential of clinical use of NK cells (Miller 2005, Rubnitz 2010, Miller 2013, Childs, Berg 2013).

Use of donor NK cells for patients ("allogeneic use") requires cell separation methods to separate wanted NK cell effects from non-wanted, contra-indicatory effects of non-NK cells, e.g. T cells. Those methods are well established (Leung 2014) and can be automated within closed, sterile systems (Apel 2013).

The maximal NK cell dose that can be obtained from a donor's leukapheresis harvest is limited to around 2x10⁸ purified NK cells. Beneficial effects of NK cell infusions into patients are dose dependent, expecting a superior clinical outcome of patients receiving higher NK cell numbers. Target cell numbers are 2x10⁹ to 1x10¹¹ NK cells to maximize the beneficial effects ofNK cell infusion.

Chimeric antigen receptors (hereinafter also abbreviated as "CAR") can direct cytotoxic immune effector cells to tumor cells. Clinical trials are performed to evaluate patient derived ("autologous") CAR T cells for clinical safety and efficacy. Modifying NK cells rather than T cells with CARs allows the use of healthy donor derived ("allogeneic") immune effector cells (Glienke 2015). The use of primary cells from a patient or donor allows for manufacturing of a clinical cell dose for a single patient. Manufacturing of cell products for multiple patients requires the use of cells that can proliferate without limitation, e.g. tumor derived cell lines. NK cell lines modified with CARs are used in clinical protocols (NK-92, Klingemann, 2014). The use of cell lines for treatment of patients requires growth arrest by irradiation to avoid continued growth of the cell line after transfer to the patient. This also reduces survival and functionality of effector cells. In addition cell lines have homogenous phenotype and function, not providing a broad reactivity against tumor cells as primary immune effector cells do.

Several protocols have been described to induce NK cell proliferation in vitro to increase the number of NK cells, hereinafter also described as "NK cell expansion" in other sources also described as "NK cell multiplication".

NK cells can be expanded in vitro by cultivating with combinations of cytokines, by supplementing cell culture media with small molecules (e.g. Nicotinamide) and by combinations of cytokines, antibodies and feeder cells. Cytokine based NK cell cultures result in only a minor increase in cell numbers that are not sufficient to manufacture NK cell products for multiple patients or from small NK cell subpopulations. NK cells stop growing in these protocols after 3 weeks, a prolonged culture does not result in higher NK cells numbers. Feeder cell based NK cell expansion protocols generate higher NK cell numbers. Effective protocols require the use of either genetically modified feeder cells (K562) or B cell lines naturally immortalized by infection with Epstein-Barr Virus. NK cells expanded in co-culture with genetically modified K562 cells (membrane bound IL-15) became senescent at 4 weeks, use of K562 cells genetically engineered to express membrane bound IL-21 continued to expand for up to 6 weeks. B cells can be immortalized by natural infection of B cells with Epstein-Barr Virus. These EBV-LCLs can be used to efficiently expand NK cells without further genetic modification of the feeder cells (Childs, Berg 2013, Denman 2012).

Long term cultures of NK cells co-cultivated with EBV-LCLs could only maintain continued growth for up to 6 months in a very small subset of NK cells (Hercend, 1982), not maintaining the initial heterogeneity in phenotype and function of the starting NK cell population and providing only limited numbers of NK cells.

Therefore there is a need for an improved in-vitro method for expanding NK cells which allows to expand NK cells to very high numbers, preferentially to numbers which are sufficient to serve clinical trials for clinical application of the expanded NK cells in patients.

### Summary of the invention

Surprisingly, it was found that NK cells can be expanded (proliferated) in cell culture systems to very high numbers within weeks of cultivation, e.g. to a 1x10¹¹-fold expansion ofNK cells in 7 weeks with a standard starting concentration of NK cells in a cell culture medium comprising a population of 2.5x10⁴ NK cells/mL or 5.25x10⁵ total cells/mL (including feeder cells). The amounts of cells achieved by such 1x10¹¹-fold expansion ofNK cells are sufficient for clinical applications such as cell therapy, e.g. adoptive immunotherapy, for leukemia and other cancers. Unexpectedly, the adding of an effective concentration of interleukin-21 (IL-21) to a cell culture medium suitable for expanding NK cells at the beginning of the cultivation process of the NK cells in combination of the presence of feeder cells which are B cell derived and EBV transformed such as Epstein-Barr virus-transformed lymphoblastoid cell lines (EBV-LCL) and the repeated addition of fresh feeder cells which are B cell derived and EBV transformed such as EBV-LCL during the whole duration of cultivation of NK cells, result in a very high number of expanded NK cells. Preferentially fresh feeder cells are added to the medium once every 5^{th} to 16^{th} day after the previous addition of said feeder cells, more preferentially once every 6^{th} to 14^{th} day after the previous addition of said feeder cells, most preferentially every 13^{th} day after the previous addition of said feeder cells. A repeated addition of IL-21 at later time points of the cultivation process or even the permanent presence of IL-21 in said cell culture medium is counterproductive for the NK cell expansion process.

It is surprising that under the conditions disclosed herein the expansion of NK cells can be proceeded over many weeks without loss of increase of NK cell numbers (there is a correlation of NK cell number over the duration of time). The duration of expansion may be proceeded (maintained) over e.g. 5,10,15, 20, 25, 30 or more weeks.

The starting concentration of NK cells in a cell culture medium comprising a population of NK cells may be between 1 cell/ mL and 2x10⁷ cells/mL, preferentially between 20 cells/mL and 2x10⁷ cells/mL. More surprisingly, it was found that the method disclosed herein results in especially high expansion of NK cells when the starting concentration of NK cells/mL in a cell culture medium comprising a population of NK cells is between 1 cell/ mL and 2.5x10⁴ cells/mL, preferentially between 20 cells/mL and 2.5x10⁴ cells/mL, i.e. the starting concentration of NK cells is lower than the standard starting concentration of NK cells which normally is higher than 7x10⁴ NK cells per mL in common NK cell expansion protocols. This is unexpected since a critical cell density is usually required to enable in vitro expansion of NK cells because NK-to-NK interactions assure survival, activation and proliferation of NK cells (Kim 2014).

The process for expansion of NK cells as disclosed herein can be fully implemented as an automated process, preferentially in a closed system under GMP conditions.

### Brief description of the drawings

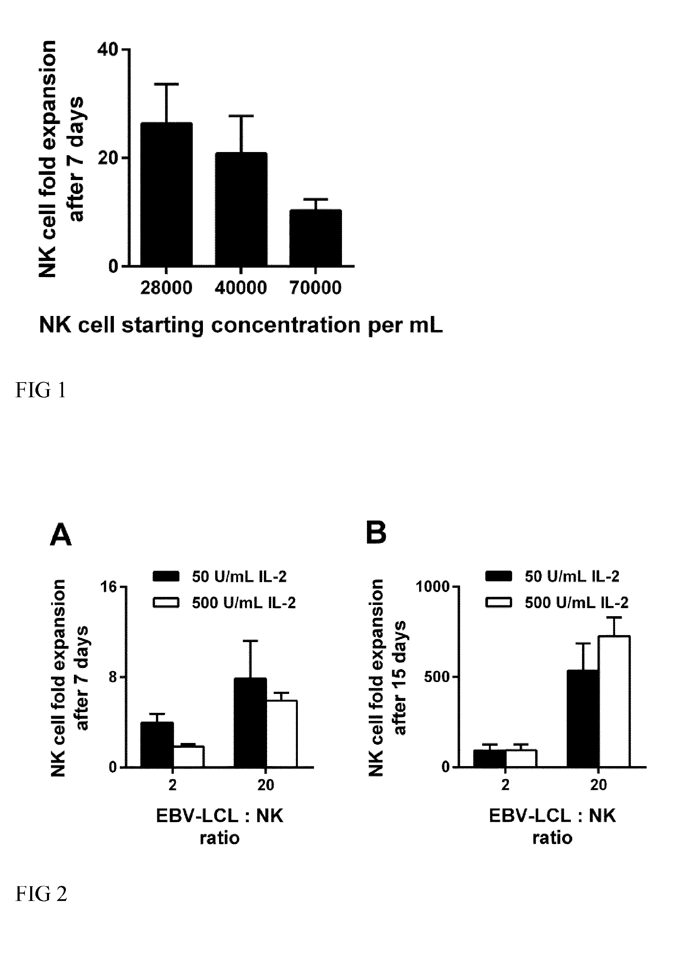

FIG 1: Impact of the NK cell starting concentration on NK cell expansion in the presence of EBV-LCL
FIG 2: feeder cell mediated expansion of NK cells at different IL-2 concentrations depending on the feeder-to-NK cell ratio:It is shown that NK cells expansion in co-culture with EBV-LCL was achieved at different IL-2 concentrations. Furthermore, higher ratios of EBV-LCL to NK cells resulted in higher NK cell expansion proofing the induction of NK cell expansion by EBV-LCL in a dose dependent manner.

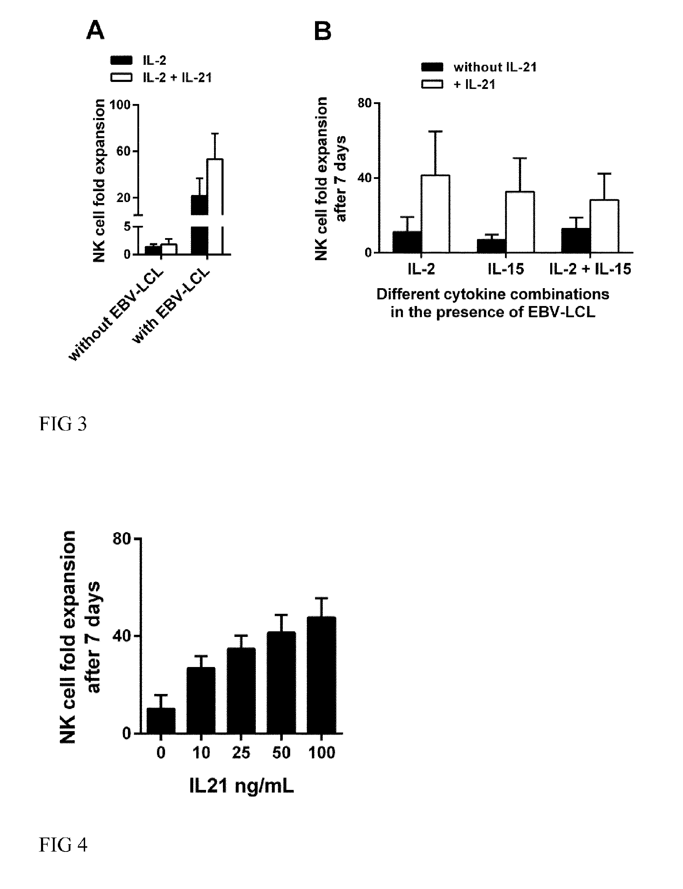

FIG 3: Effect of different cytokine combinations on feeder cell mediated NK cell expansion: It is shown that IL-2 and/or IL-15 could be applied to expand NK cells in co-culture with EBV-LCL. In addition, the EBV-LCL mediated NK cell expansion could be significantly increased by additionally using IL-21.
FIG 4: Feeder cell mediated NK cell expansion at different concentrations of IL-21: EBV-LCL mediated NK cell expansion could be increased by IL-21 in a dose dependent manner.

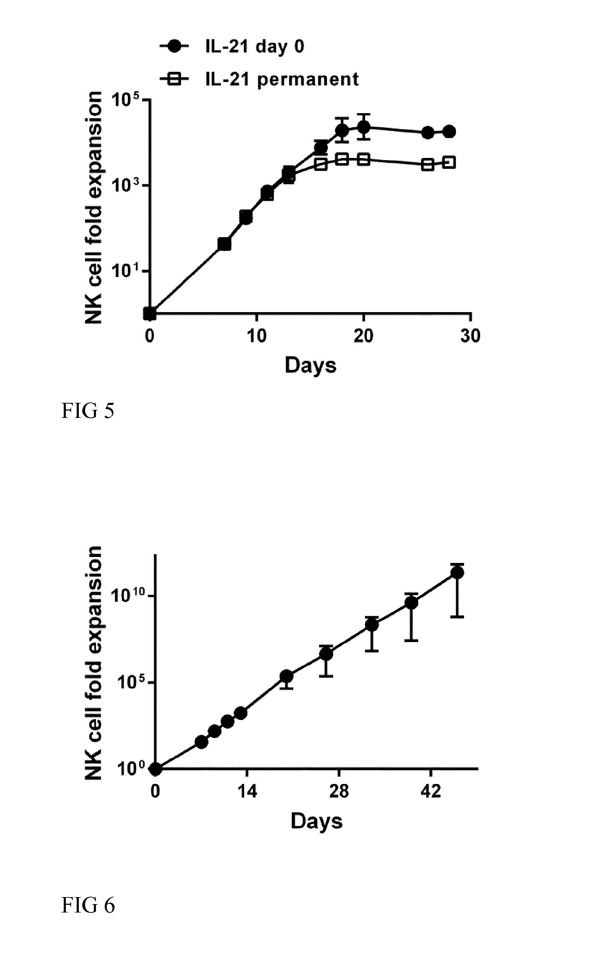

FIG 5: Effect of permanent versus short term adding of IL-21on expansion of NK cells in the presence of B cell derived feeder cells: To increase the NK cell expansion by use of IL-21, it was sufficient to add IL-21 only at the beginning of the culturing process. Surprisingly, permanent adding of IL-21 was even counteracting since it led to an inhibited long term NK cell expansion.
FIG 6: Constant long term NK cell expansion by adding IL-21 at day 0 and repeated stimulation with b cell derived feeder cells: It was possible to maintain a constant expansion of NK cells at a very high level over long time by adding IL-21 only at the beginning of cultivation in combination with repeated stimulation of the NK cells with B cell derived feeder cells.

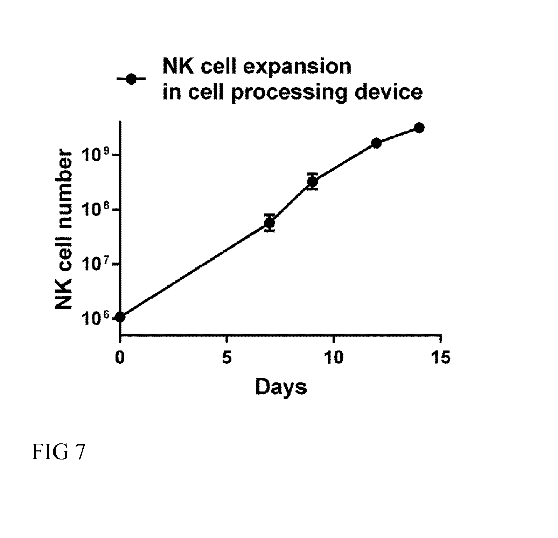

FIG 7: Expansion of NK cells within a closed system using a device for automated cell processing: Efficient production of NK cells could be realized within a closed and automated system for cell processing by applying the optimized method for NK cell expansion, that is characterized by adding IL-21 only at the beginning of cultivation and stimulation of the NK cells with B cell derived feeder cells.

### Detailed description of the invention

In a first aspect the present invention provides a method for in-vitro culturing and expanding natural killer (NK) cells in a cell culture medium comprising a population of NK cells, the method comprising
a) adding an effective concentration of interleukin-21 (IL-21) at the beginning of the culturing process to said medium
b) adding repeatedly an effective concentration of interleukin-2 (IL-2) and/or interleukin-15 (IL-15) to said medium
c) adding repeatedly feeder cells or membrane particles thereof to said medium, wherein said feeder cells are B cell derived which are EBV immortalized; and
wherein said expansion of NK cells in said cell culture medium is maintained for at least 3 weeks.

The addition of an effective concentration of IL-21 at the beginning of the culturing process has the effect that a long duration of expansion of NK cells is possible resulting in extremely high NK cell numbers. In contrast thereto a repeated addition of IL-21 at later time points or even permanent presence of IL-21 in the cell culture medium during the culturing process result in a reduction of expansion rate (see example 5 and FIG 5).

The long term NK cell expansion as disclosed herein bases on the surprising effect that the addition of said IL-21 at the beginning of the culturing process and the use of B cell derived, EBV immortalized feeder cells such as SMI-EBV-LCL which are added repeatedly to said medium results in very high expansion rates. Repeated adding of the feeder cells is required since the feeder cells rapidly disappear since they are growth inactivated, e.g. by irradiation. Instead of the feeding cells membrane particles thereof may also be used.

Il-2 and/or IL-15 are known cytokines which stimulate NK cell expansion in a cell culture. They have to be present in the medium but may be replaced by other cytokines or growth factors which are able to stimulate NK cells in an in-vitro cell culture to a basic expansion level (e.g. 10- to 50-fold expansion in two weeks). The adding of IL-21 and the presence of B cell derived feeder cells in a cell culture medium which otherwise would be able to expand the NK cells to a basic level only are primarily relevant for the effect of long term NK cell expansion as disclosed herein. Therefore, the process disclosed herein should work also with other stimulants than IL-2 and/or IL-15 for NK cell expansion which are responsible for basic NK cell expansion.

In one embodiment of the invention the effective concentration of IL-21 in said cell culture medium may be between 0.1 and 1000 ng/mL, preferentially between 1 and 200 ng/mL, more preferentially between 10 and 100 ng/mL.

In one embodiment of the invention said IL-21 is soluble IL-21 or substrate-bound IL-21.

In one embodiment of the invention the effective concentration of IL-2 in said cell culture medium may be between 1 U/mL and 5000 U/mL, preferentially between 10 U/mL and 1000 U/mL, more preferentially between 50 and 500 U/mL.

In another embodiment of the invention the effective concentration of IL-15 in said cell culture medium may be between 0.1 and 1000 ng/mL, preferentially between 1 and 200 ng/mL, more preferentially between 10 and 100 ng/mL.

In a further embodiment of the invention said IL-2 and IL-15 are added both to said cell culture medium in said effective concentrations.

In one embodiment of the invention said adding repeatedly feeder cells to said medium may be performed between day 5 and day 16 after the previous feeder cell addition, preferentially between day 6 and day14, more preferentially every 13^{th} day of the cultivation process. The concentration of feeder cells may be between 1x10⁴/mL to 1x10⁷/mL.

Preferentially, the ratio of NK cells to said feeder cells is between 1:100 and 1:1, more preferentially between 1:30 and 1:10, most preferentially 1:20.

In one embodiment of the invention said expansion of NK cells in said cell culture medium may be maintained for at least 5 weeks, preferentially for at least 7 weeks.

In one embodiment of the invention said NK cells in a cell culture medium comprising a population of NK cells may be purified NK cells. NK cells can be purified from a sample comprising NK cells and other cells, such as e.g. PMBC or whole blood, by magnetic cell separation methods such as MACS^{®} (Miltenyi Biotec) or flow cytometry methods such as FACS™. These and other methods for purification of cells, e.g. NK cells, are well known in the art.

In one embodiment of the invention the starting concentration of said NK cells in a cell culture medium comprising a population of NK cells may be between 20 cells/ mL and 2x10⁷ cells/mL.

In one embodiment of the invention the starting concentration of said NK cells in a cell culture medium comprising a population of NK cells may be between 20 cell/ mL and 2.5x10⁴ cells/mL.

In one embodiment of the invention said feeder cells may be genetically non-modified cells with exception of the introduction of the EBV genome as a result of immortalization of said feeder cells.

In one embodiment of the invention said NK cells in a cell culture medium comprising a population of NK cells may be genetically modified. Said genetically modified NK cells may express a chimeric antigen receptor (CAR).

In one embodiment of the invention said method is performed in an automated process. Said process may be performed in a closed system.

In a further aspect the present invention provides a a pharmaceutical composition comprising a population of NK cells produced according to the method disclosed herein.

Said composition can be homogeneous regarding phenotype and function. The desired phenotype and functional characteristics can be selected from the heterogeneous starting population by pre-selection or cloning methods..

In general, the expanded NK cells achieved with the method of the present invention can be used in subsequent therapeutic or non-therapeutic applications.

Target cell populations, such as the NK cell populations obtained via the present disclosure may be administered either alone, or as a pharmaceutical composition in combination with diluents and/or with other components such as IL-2 or other cytokines or cell populations. Briefly, pharmaceutical compositions of the present disclosure may comprise a target cell population as described herein, in combination with one or more pharmaceutically or physiologically acceptable carriers, diluents or excipients. A pharmaceutical composition may comprise
a) a population of NK cells, wherein said NK cells are proliferated to therapeutically effective amounts according to the present invention; and b) one or more pharmaceutically or physiologically acceptable carriers, diluents or excipients.

Compositions of the present disclosure are preferably formulated for intravenous administration.

Pharmaceutical compositions of the present disclosure may be administered in a manner appropriate to the disease to be treated (or prevented). The quantity and frequency of administration will be determined by such factors as the condition of the patient, and the type and severity of the patient's disease, although appropriate dosages may be determined by clinical trials.

### Definitions

Unless defined otherwise, technical and scientific terms used herein have the same meaning as commonly understood by one of ordinary skill in the art to which this invention belongs.

The term "natural killer cells (NK cells)" are defined as large granular lymphocytes (LGL) and constitute the third kind of cells differentiated from the common lymphoid progenitor-generating B and T lymphocytes. NK cells are known to differentiate and mature in the bone marrow, lymph nodes, spleen, tonsils, and thymus, where they then enter into the circulation. NK cells differ from natural killer T cells (NKTs) phenotypically, by origin and by respective effector functions; often, NKT cell activity promotes NK cell activity by secreting IFNγ. In contrast to NKT cells, NK cells do not express T-cell antigen receptors (TCR) or pan T marker CD3 or surface immunoglobulins (Ig) B cell receptors, but they usually express the surface markers CD16 (FcγRIII) and CD56 in humans, NK1.1 or NK1.2 in C57BL/6 mice. Up to 80% of human NK cells also express CD8.

As used herein the term "culturing" includes providing the chemical and physical conditions (e.g., temperature, gas) which are required for NK cell maintenance, and growth factors. Often culturing the NK cells includes providing the NK cells with conditions for expansion (proliferation). Examples of chemical conditions which may support NK cell expansion include but are not limited to buffers, serum, nutrients, vitamins, antibiotics, cytokines and other growth factors which are regularly provided in (or may be given manually to) the cell culture medium suited for NK cell expansion. In one embodiment, the NK cell culture medium includes TexMACS Research Medium (Miltenyi Biotec GmbH) supplemented with 5% human serum type AB (Life Technologies) and 500 U/mL of IL-2 (Proleukin S, Novartis). In another embodiment the NK cell culture medium includes Stem Cell Growth Medium SCGM (Cell Genix) supplemented with 5% human serum type AB (Life Technologies) and 500 U/mL of IL-2 (Proleukin S, Novartis). Other media suitable for use to expand NK cells are well known in the art.

The term "cell culture medium" as used herein includes liquids providing the chemical conditions which are required for NK cell maintenance. Examples of chemical conditions which may support NK cell expansion include but are not limited to solutions, buffers, serum, serum components, nutrients, vitamins, cytokines and other growth factors which are regularly provided in (or may be given manually to) the cell culture medium. Media suitable for use to cultivate NK cells as known in the art include TexMACS (Miltenyi), CellGro SCGM (CellGenix), X-Vivo 10, X-Vivo 15, BINKIT NK Cell Initial Medium (Cosmo Bio USA), AIM-V (Invitrogen), DMEM/F12, NK Cell Culture Medium (upcyte technologies).

The term "effective concentration of IL-21" as used herein is defined as that final concentration of IL-21 or derivatives thereof in the cell culture medium which, when provided to the population of NK cells in cell culture, preferentially at day 0 of the culturing process, results in a greater expansion of the NK cells, as compared to NK cells cultured under identical conditions but without provision of the IL-21 to the population of NK cells. IL-21 concentrations suitable for use in some embodiments of the present invention are typically in the range between 0.1 and 1000 ng/mL, preferentially between 1 and 200 ng/mL, preferentially between 10 and 100 ng/mL. The Examples below demonstrate exemplary effective concentrations of the IL-21. Preferentially, the IL-21 is soluble or substrate-bound. It would be unfavorable if the IL-21 is membrane-bound on the feeder cells (e.g. on genetically modified feeder cells that express IL-21 on the cell surface) as both components the IL-21 and the feeder cells could not be added separately to the cell culture which is a prerequisite for the performance of the expanding process as disclosed herein.

The terms "IL-2" and "IL-15" as used herein refer to cytokines Interleukin-2 and Interleukin-15 and derivatives thereof e.g. IL-2 superkine, IL-2 diphteria toxin fusion protein or IL-15Rα sushi.

The term IL-2 and/or IL-15 generally refers to members of the 4α-helix bundle family of cytokines binding to the heterotrimeric receptors for IL2 and IL15 sharing the common gamma chain and IL2/IL15Rβ (also known as IL2Rβ, CD122).

The term "adding repeatedly an effective concentration of IL-2 and/or IL-15" as used herein refers to concentrations of IL-2 in said cell culture medium between 1 U/mL and 5000 U/mL, preferentially between 10 U/mL and 1000 U/mL, more preferentially between 50 and 500 U/mL, and/or refers to concentrations of IL-15 in said cell culture medium between 0.1 and 1000 ng/mL, preferentially between 1 and 200 ng/mL, more preferentially between 10 and 100 ng/mL. The Examples below demonstrate exemplary effective concentrations of IL-2 and/or IL-15. Normally, the concentration of IL-2 and/or IL-15 decreases with time during the culturing process, therefore IL-2 and/or IL-15 is added repeatedly to the cell culture medium to maintain the levels of the cytokine(s) at the effective concentration(s). Regularly, IL-2 and/or IL-15 is added again to the cell culture by a cell culture medium change. In this context "repeatedly" means at least one repeat within the whole culturing process.

As used herein, the term "expansion" or "proliferation" refers to cell growth and multiplication of cell numbers. Expansion or proliferation, as used herein relate to increased numbers of NK cells occurring during the cultivation process as e.g. disclosed herein.

The terms "culturing process" or "cultivation" as used herein refer to the culturing and expansion of NK cells as disclosed herein, wherein the starting day (starting point) of the culturing process, i.e. the expansion of NK cells, is defined as day 0. The culturing process may last as long as desired by the operator and can be performed as long as the cell culture medium has conditions which allow the cells to survive and/or grow and/or proliferate.

The term "beginning of the culturing process" as used herein refers to the start of the expanding process by adding growth factors such as IL-2 and/or IL-15 to the cell culture medium, i.e. the cells are initiated to start their proliferation process. The phase of the beginning of the cultivation process comprise the first three days since exposure of NK cells to such growth factors. Within this time period, i.e. within the first three days, it is advantageous to add the effective concentration of IL-21 to said medium. Later time points for adding the IL-21 to said medium have not the beneficial effect compared to
the adding at the earlier time points. Therefore, the addition of an effective concentration of interleukin-21 (IL-21) at the beginning of the culturing process to said medium means that said IL-21 is added within the first three days of the culturing process, preferentially at day 0 of the culturing process.

Preferentially, the effective concentration of IL-21 is added once to the cell culture medium only. Alternatively, the effective concentration of IL-21 is added repeatedly, e.g. twice or more frequently, to said cell culture medium but within the first three days of the cultivation process ("at the beginning"). Said twice or more frequently addition of IL-21 to the medium may also be organized in such a way that the effective concentration of IL-21 in the cell culture medium reaches the effective concentration not before the sum of said several additions.

As mentioned above, an addition of IL-21 to said cell culture medium later than at the beginning phase of the cultivation process is counterproductive to the outcome of the 10¹¹-fold expansion of NK cells obtainable within 7 weeks by the method of the present invention. Especially counterproductive is the permanent presence of IL-21 in the cell culture medium during the culturing process.

The term "feeder cells" refers to cells that are added to a culture of target cells (i.e. here Natural Killer cells) to support their survival and/or growth. Feeder cells provide an intact and functional extracellular matrix and matrix-associated factors and secrete known and unknown cytokines into the conditioned medium. Feeder cells are usually growth arrested to prevent their proliferation in the culture, but their survival is maintained. Growth arrest can be achieved by irradiation with an effective dose or treatment with an effective dose of chemicals such as Mytomycin C. There are several kind of feeders cells including irradiated Peripheral Blood Mononuclear cells (hereinafter also abbreviated as "PBMC"), PBMCs depleted of NK cells, cancer cell lines, genetically engineered cancer cell lines and lymphocytes immortalized by natural infection with Epstein-Barr Virus (hereinafter also abbreviated as "EBV").

The term "membrane particles of feeder cells" as used herein refers to membrane preparations of the feeder cells that contain the NK cell stimulating surface molecules of the feeder cells. Membrane particles of the feeder cells may be utilized to avoid possible disadvantages of live feeder cells. Membrane particles of feeder cells can be produced by lysis and disruption of the cells using nitrogen cavitation followed by isolation and purification of the cell membrane fraction by density gradient centrifugation, exemplary described by Oyer et al. (Oyer 2015). Normally, in the method disclosed herein the feeder cells can be replaced by membrane particles of said feeder cells. Preferentially, the membrane particles should be used in such amounts that is comparable to these amounts that can be obtained by the used live feeder cells. Preferentially the concentration of used membrane particles may be between 100 and 400 µg/mL, more preferentially between 150 and 250 µg/mL. The addition of membrane particles may be performed as frequently as the addition of live feeder cells.

EBV-LCL as used herein can be produced e.g. by immortalization of B cells by EBV. PBMC are cultured with Cyclosporin A and the EBV laboratory strain B95-8. The cells are cultured until clear proliferation could be observed. After maintaining the cells in culture containing Acyclovir to prevent release of infectious virus the generation of a EBV transformed lymphoblastoid cell line is completed. Further protocols for producing Epstein-Barr virus-transformed lymphoblastoid cell lines (EBV-LCL) are well known in the art and disclosed e.g. in Sili et al, 2012, Cytotherapy, 14(1):7-11(SOP 3 in supplementary material).

The terms "engineered cell" and "genetically modified cell" as used herein can be used interchangeably. The terms mean containing and/or expressing a foreign gene or nucleic acid sequence which in turn modifies the genotype or phenotype of the cell or its progeny. Especially, the terms refer to the fact that cells can be manipulated by recombinant methods well known in the art to express stably or transiently peptides or proteins which are not expressed in these cells in the natural state. Genetic modification of cells may include but is not restricted to transfection, electroporation, nucleofection, transduction using retroviral vectors, lentiviral vectors, non-integrating retro- or lentiviral vectors, transposons, designer nucleases including zinc finger nucleases, TALENs or CRISPR/Cas.

The term " said feeder cells are genetically non-modified cells with exception of the introduction of the EBV genome as a result of immortalization of said feeder cells" as used herein means that the feeders cells only have been manipulated by natural EBV infection to become immortalized but no further genetic engineering (genetic modification) has been involved resulting feeder cells which do not bear additional genetic information with exception of the endogenous genetic material of the feeder cells themselves and the genetic information of the natural Epstein-Barr Virus (EBV).

The term "closed system" as used herein refers to any closed system which reduces the risk of cell culture contamination while performing culturing processes such as the introduction of new material and performing cell culturing steps such as proliferation, differentiation, activation, and/or separation of cells. Such a system allows to operate under GMP or GMP-like conditions ("sterile") resulting in cell compositions which are clinically applicable. Herein exemplarily the CliniMACS Prodigy® (Miltenyi Biotec GmbH, Germany) is used as a closed system. This system is disclosed in WO2009/072003. But it is not intended to limit the use of the method of the present invention to the CliniMACS® Prodigy.

The terms "automated method" or "automated process" as used herein refer to any process being automated through the use of devices and/or computers and computer softwares which otherwise would or could be performed manually by an operator. Methods (processes) that have been automated require less human intervention and less human time to deliver. In some instances the method of the present invention is automated if at least one step of the present method is performed without any human support or intervention. Preferentially the method of the present invention is automated if all steps of the method as disclosed herein are performed without human support or intervention. Preferentially the automated process is implemented on a closed system such as CliniMACS® Prodigy.

CARs comprise a single chain fragment variable (scFv) of an antibody specific for a certain target antigen coupled via hinge and transmembrane regions to cytoplasmic domains of cell signaling molecules. The most common lymphocyte activation moieties include a cell co-stimulatory (e.g. CD28, CD137, OX40, ICOS, and CD27) domain in tandem with a cell triggering (e.g. CD3ζ) moiety. The CAR-mediated adoptive immunotherapy allows CAR-grafted cells to directly recognize the desired antigen on target cells in a non-HLA-restricted manner.

### Embodiments

In one embodiment of the invention NK cells are purified from a human blood sample such as PBMC by positive magnetic cell separation. The NK cells are separated by use of magnetic beads coupled to an anti-CD56 antibody or fragment thereof. The positive fraction comprising an enriched population of NK cells is then added to a cell culture medium suitable for expansion of NK cells, i.e. the medium comprises Il-2 and/or IL-15 and B-cell derived feeder cells such as EBV-LCL. To begin the culturing process IL-21 is added to the medium at day 0. The cells are cultivated at 37°C and 5% CO₂. After 5 or 7 days, fresh medium comprising IL-2 and/or IL-15 is added the first time. Thereafter, fresh medium comprising IL-2 and/or IL-15 is added every second to fifth day. Every 13th day of the cultivation process, fresh B cell derived feeder cells are added to the medium.

This process described above is performed as long as required to reach the desired amount of expanded NK cells and the produced NK cells can be harvested.

In one embodiment of the invention said NK cells in a cell culture medium comprising a population of NK cells may be NK cells which are purified from a human blood sample by removal of T cells by use of magnetic beads coupled to an anti-CD3 antibody or fragment thereof and further purification by CD56 enrichment.

In one embodiment of the invention said NK cells in a cell culture medium comprising a population of NK cells may be NK cells which are purified by CD3 depletion and CD56 enrichment using a clinical scale cell separator (CliniMACS plus, Miltenyi Biotec, Germany). In one embodiment of the invention said NK cells in a cell culture medium comprising a population of NK cells may be purified by CD3 depletion and CD56 enrichment including all magnetic labeling steps in a completely closed system (CliniMACS Prodigy^{®}, Miltenyi Biotec, Germany).

In one embodiment of the invention said NK cells in a cell culture medium comprising a population of NK cells may be NK cells which are purified from a human blood sample such as PBMC by negative magnetic cell separation. The NK cells are separated by use of magnetic beads coupled to a cocktail of antibodies or fragment thereof binding to non-NK cells. The negative fraction comprising an enriched population of NK cells is then added to a cell culture medium suitable for expansion of NK cells, i.e. the medium comprises Il-2 and/or IL-15 and B-cell derived feeder cells such as EBV-LCL.

In one embodiment of the invention said NK cells in a cell culture medium comprising a population of NK cells may be NK cells which are purified from whole human blood by negative magnetic cell separation and erythrocyte sedimentation as dislosed e.g. in WO2013076070A1 (MACSxpress^{®} NK cell isolation kit, Miltenyi Biotec).

In one embodiment of the invention said NK cells in a cell culture medium comprising a population of NK cells may be NK cells which are purified subpopulations from negatively selected NK cells.

In one embodiment of the invention said NK cells in a cell culture medium comprising a population of NK cells may be NK cells which are purified NKG2C positive NK cell subpopulations.

In one embodiment of the invention said NK cells in a cell culture medium comprising a population of NK cells may be NK cells which are purified CD57 positive and NKG2C positive adaptive NK cell subpopulations.

In one embodiment of the invention said NK cells in a cell culture medium comprising a population of NK cells may be NK cells which are purified NK cell subpopulations positive for single KIR molecules.

In one embodiment of the invention said NK cells in a cell culture medium comprising a population of NK cells may be NK cells which are purified NK cell subpopulations that are specific for virus peptides.

In one embodiment of the invention said NK cells in a cell culture medium comprising a population of NK cells may be NK cells which are enriched for a cytokine secreting subpopulation.

In one embodiment of the invention said NK cells in a cell culture medium comprising a population of NK cells may be NK cells which are isolated subpopulations using a fluorescence activated cell sorter.

In one embodiment of the invention said NK cells in a cell culture medium comprising a population of NK cells may be NK cells which are isolated subpopulations using a microchip based cell sorter such as MACSQuant Tyto^{®} (Miltenyi Biotec GmbH)..

In one embodiment of the invention said NK cells in a cell culture medium comprising a population of NK cells may be NK cells which are genetically modified. Said genetically modified NK cells may express a chimeric antigen receptor (CAR).

In one embodiment of the invention said NK cells in a cell culture medium comprising a population of NK cells may be NK cells which are genetically modified. Said genetically modified NK cells may overexpress one or multiple chemokine receptors.

In one embodiment of the invention said NK cells in a cell culture medium comprising a population of NK cells may be NK cells which are genetically modified. Said genetically modified NK cells may express a higher affinity version of NK cell receptors than naturally occurring. Said NK cell receptors may be selected from the group of CD16, CD314/NKG2D, CD335/NKp46, NKp44, NKp30, CD226/DNAM-1 or activating Killer Immunoglobulin like receptors.

In one embodiment of the invention the method consisting of NK cell isolation and NK cell cultivation is performed in a closed system as known by the person skilled in the art. In a further embodiment the closed system is a disposable tubing set attached to a cell processing device. In a further embodiment that said cell processing device is a CliniMACS Prodigy^{®} (Miltenyi Biotec).

In one embodiment of the invention said culturing process is used for industrial manufacturing of NK cells in large bioreactors. In a further embodiment of the invention NK cells are isolated from an apheresis product and these said about 2x10⁸ donor NK cells are cultivated with said culturing process for 3 weeks, resulting in about 2x10¹³ NK cells in a volume of about 200 to 1000 liters. In a further embodiment of the invention those about 2x10¹³ NK cells are split into fractions of 1x10¹¹ NK cells each, providing clinical NK cell products for about 200 patients.

In a further embodiment of the invention NK cells are isolated from 100 mL of a donation of human whole blood and these said about 2x10⁷ donor NK cells are cultivated with said culturing process for 4 weeks, resulting in about 2x10¹⁴ NK cells in a volume of about 2000 to 10000 liters. In a further embodiment of the invention those about 2x10¹⁴ NK cells are split into fractions of 1x10¹¹ NK cells each, providing clinical NK cell products for about 2000 patients.

In a further embodiment of the invention NK cells are isolated from 1 mL of a donation of human whole blood and these said about 2x10⁵ donor NK cells are cultivated with said culturing process for 6 weeks, resulting in about 2x10¹⁴ NK cells in a volume of about 3000 to 15000 liters. In a further embodiment of the invention those about 2x10¹⁴ NK cells are split into fractions of 1x10¹¹ NK cells each, providing clinical NK cell products for about 2000 patients.

In a further embodiment of the invention a single NK cell is isolated from a donation of human whole blood and this said NK cell is cultivated with said culturing process for 11 weeks, resulting in about 1x10¹⁵ NK cells in a volume of about 10000 to 50000 liters. In a further embodiment of the invention those about 1x10¹⁵ NK cells are split into fractions of 1x10¹¹ NK cells each, providing clinical NK cell products for about 10000 patients.

In one embodiment of the invention said NK cells are cultivated in said culturing process for 13 days, 99.9% of the cultivation volume is harvested and 0.1% of the volume is further introduced in said culturing process. Harvested NK cells are used for desired purposes and said culturing process provides a continuous supply of about 1x10⁹ NK cells every two weeks. In a further embodiment of the invention said cultivated NK cells are cryopreserved after two weeks. Aliquots of cryopreserved NK cells are thawed and further cultivated in said culturing process, allowing for NK cell production starting from a cryopreserved cell bank.

In one embodiment of the invention said NK cells are cloned by limiting dilution, exemplary described by Morris et al. (Morris 2005) to isolate a desired phenotype of said NK cells for introduction to said culturing process.

In one embodiment of the invention said feeder cells are selected for a desired HLA phenotype for modulation of the KIR repertoire of the NK cells in said culturing process.

### Examples

### Example 1: Impact of the NK cell starting concentration on NK cell expansion in the presence of B cell derived feeder cells

Efficient methods for NK cell expansion are needed to generate sufficient NK cells for NK cell based immunotherapy. In this context, it is known that NK cells require homotypic NK-to-NK interactions in culture for optimal survival, activation and proliferation (Kim 2014), and, in line with this observation, it is well known that maintenance of a critical cell density is required for optimal in vitro expansion of NK cells.

Surprisingly, it was found that very low NK cell densities provoke an improved NK cell expansion in the presence of B cell derived feeder cells. Human peripheral blood mononuclear cells (PBMC) were prepared from buffy coat preparations from human whole blood. Immortalization of B cells by EBV was used to generate B cell derived feeder cells. PBMC were cultured with Cyclosporin A and the EBV laboratory strain B95-8. The cells were cultured until clear proliferation could be observed. After maintaining the cells in culture containing Acyclovir to prevent release of infectious virus the generation of the EBV transformed lymphoblastoid cell line (EBV-LCL) was completed. The EBV-LCL line used in all examples was SMI-EBV-LCL. NK cells were isolated from PBMC using the NK cell isolation kit (Miltenyi Biotec). NK cells from three different donors were cultivated in TexMACS Research Medium (Miltenyi Biotec) supplemented with 5% human serum type AB (Life Technologies) and 500 U/mL of IL-2 (Proleukin S, Novartis), together with 100 Gy irradiated EBV-LCL (SMI-EBV-LCL) at a ratio of 1:20. Different NK cell concentrations were investigated to initiate the cultivation, while the NK-to-feeder cell ratio was kept constant by changing not solely the concentrations of NK cells but changing the concentration of the total cells. The cells were cultivated at 37°C and 5 % CO₂ and the expansion of NK cells was determined after 7 days. The NK cell fold expansion is calculated as NK cell number at day 7 divided by NK cell number at start of cultivation. As shown in FIG 1, NK cell starting concentrations of 2.8x10⁴ NK cells/mL, 4.0x10⁴ NK cells/mL and 7.0x10⁴ NK cells/mL resulted in 26.4, 20.9 and 10.3 mean NK cell fold expansion respectively. Error bars in FIG 1 are for standard deviation of the different donors.

It could be clearly shown that NK cells expanded efficiently in co-culture with B cell derived feeder cells if the cultivation was initiated at extremely low NK cell concentrations and, surprisingly, lower starting NK cell concentrations led to significantly higher expansion of NK cells.

### Example 2: feeder cell mediated expansion of NK cells at different IL-2 concentrations and the use of different feeder-to-NK cell ratios

In order to investigate the effect of the NK-to-feeder cell ratio and different concentrations of IL-2 on the NK cell expansion, human NK cells from three different donors were cultivated in TexMACS Research Medium supplemented with 5% human serum type AB together with 100 Gy irradiated EBV-LCL (SMI-EBV-LCL). The used concentration of total cells was 5.25x10⁵/mL including NK cells and EBV-LCL. Different ratios of NK cell to EBV-LCL were tested and ratios of 1 to 2 and 1 to 20 are shown in FIG 2. For each NK-to-feeder ratio different concentrations of IL-2 were used in the medium and 50 U/mL and 500 U/mL are shown in FIG 2. The cells were cultivated at 37°C and 5 % CO₂. At day 5, 7, 10, and 12 the NK cell concentration was determined and the NK cell concentration was diluted to 5x10⁵/mL by adding fresh medium containing IL-2. The NK cell fold expansion is calculated as NK cell number at the day of interest divided by NK cell number at start of cultivation. The mean values for NK cell fold expansion are displayed for day 7 (FIG 2A) and day 15 (FIG 2B). Error bars in FIG 2 are for standard deviation of the different donors. At day 7 of cultivation, use of 50 U/mL IL-2 resulted in 4.0 and 7.9 mean NK cell fold expansion for the 1:2 and 1:20 NK-to-feeder ratios while use of 500 U/mL IL-2 resulted in 1.9 and 5.9 mean fold expansion for the 1:2 and 1:20 NK-to-feeder ratios. At day 15 of cultivation, use of 50 U/mL IL-2 resulted in 93.6 and 535.6 mean NK cell fold expansion for the 1:2 and 1:20 NK-to-feeder ratios while use of 500 U/mL IL-2 resulted in 94.4 and 726.1 mean fold expansion for the 1:2 and 1:20 NK-to-feeder ratios. In conclusion, for feeder cell mediated NK cell expansion it was possible to use different IL-2 concentrations, different NK to EBV-LCL cell ratios could be applied and higher numbers of EBV-LCL per NK cell led to enhanced expansion of NK cells showing that the induction of increased NK cell expansion was based on EBV-LCL in a dose dependent manner.

### Example 3: Effect of different cytokine combinations on feeder cell mediated NK cell expansion

Human donor derived NK cells were cultivated in TexMACS Research Medium supplemented with 5% human serum type AB together with or without 100 Gy irradiated EBV-LCL (SMI-EBV-LCL) at a ratio of 1:20 and a starting concentration of 5.25x10⁵/mL total cells. In addition, 500 U/mL IL-2 and/or 10 ng/mL IL-15 (Miltenyi) were tested as medium supplements together with or without adding of 100 ng/mL IL-21 (Miltenyi). The cells were cultivated at 37°C and 5 % CO₂ and the expansion of NK cells was determined after 7 days. For different cytokine combination the mean value for NK cell fold expansion is displayed in FIG 3. The NK cell fold expansion is calculated as NK cell number at day 7 divided by NK cell number at start of cultivation. Error bars in FIG 3 are for standard deviation of different donors. Results from eight different donors can be seen in FIG 3A.

NK cells did not noticeable expand without EBV-LCL independent of the use of IL-2 and IL-21. In contrast, NK cells significantly expanded in the presence of EBV-LCL and IL-2 shown by 21.8 mean NK cell fold expansion after 7 days. Surprisingly, additionally adding of IL-21 further enhanced the EBV-LCL mediated NK cell expansion in the presence of IL-2 and resulted in 53.2 mean NK cell fold expansion after 7 days. In FIG 3B the data from six different donors are shown and co-culture of NK cells with EBV-LCL and using IL-2, IL-15 or a combination of IL-2 and IL-15 resulted in 11.0, 6.9 and 12.6 mean NK cell fold expansion respectively. Confirming the observation from FIG 3A, adding of IL-21 further enhanced the EBV-LCL mediated NK cell expansion and resulted in 41.4, 32.6 or 28.1 mean NK cell fold expansion in in the presence of IL-2, IL-15 or the combination of IL-2 and IL-15. In conclusion, IL-2 and IL-15 or a combination of both cytokines enabled the expansion of NK cells in the presence of B cell derived feeder cells. Surprisingly, Supplementation of IL-21 further increased the EBV-LCL based expansion of NK cells while IL-21 had no effect on the expansion of NK cells without feeder cells.

### Example 4: Feeder cell mediated NK cell expansion at different concentrations of IL-21

NK cells from three different donors were cultivated in TexMACS Research Medium supplemented with 5% human serum type AB and 500 U/mL IL-2 together with 100 Gy irradiated EBV-LCL (SMI-EBV-LCL) at a ratio of 1:20 and a starting concentration of 5.25x10⁵/mL total cells. Different concentrations of IL-21 were added at day 0. The cells were cultivated at 37°C and 5 % CO₂ and the expansion of NK cells was determined after 7 days. The NK cell fold expansion is calculated as NK cell number at day 7 divided by NK cell number at start of cultivation. In FIG 4 mean NK cell fold expansion for NK cells from three different donors are depicted and error bar are for standard deviation. Using 0, 10, 25, 50 and 100 ng/mL IL-21 resulted in 10.1, 26.8, 34.9, 41.4 and 47.7 mean NK cell fold expansion respectively, showing that there is a positive correlation of the EBV-LCL based NK cell expansion and the applied IL-21 concentration.

### Example 5: Effect of permanent versus short term adding of IL-21on expansion of NK cells in the presence of B cell derived feeder cells

It was tested weather maintaining a constant IL-21 concentration is required to assure a sustained positive effect on the NK cell expansion.

Human NK cells from three different donors were cultivated in TexMACS Research Medium supplemented with 5% human serum type AB and 500 U/mL IL-2 together with 100 Gy irradiated EBV-LCL (SMI-EBV-LCL) at a ratio of 1:20 and a starting concentration of 5.25x10⁵/mL total cells. The cells were cultivated at 37°C and 5 % CO₂. At day 7, 9, 11, 13, 16, 18, 20, 26 and 28 the NK cell concentration was determined and the NK cell concentration was diluted to 5x10⁵/mL by adding fresh medium containing IL-2. IL-21 (100 ng/mL) was either added only at the beginning of the culturing process or was supplemented permanently as component of the medium, meaning at the beginning of the and whenever fresh medium was added. The mean values for NK cell fold expansions from three different donors at different time points are shown in FIG 5 and error bars in FIG 5 are for standard deviation. The NK cell fold expansion is calculated as NK cell number at the day of interest divided by NK cell number at beginning of the cultivation. Adding IL-21 only at the beginning of the cultivation (black dots) or adding IL-21 permanently (open squares) resulted in 18383 or 3473 mean NK cell fold expansion after 28 days. In conclusion, adding IL-21only at the beginning of the culturing process was sufficient to achieve the enhanced feeder cell mediated expansion of NK cells while permanent adding of IL-21 unexpectedly had a negative effect demonstrated by a reduced long-term NK cell expansion.

### Example 6: Constant long term NK cell expansion by adding IL-21 at day 0 and repeated stimulation with b cell derived feeder cells

Taken together, in example 1-5 it is shown that cultivating NK cells together with B cell derived feeder cells in combination with adding IL-21 only at the beginning of the cultivation allows the expansion of NK cells with outstanding efficacy. However, since primary NK cells in general can only be expanded for a limited time period as shown in FIG 5, it was tested how to maintain the high level for a longer time period.

NK cells from six different donors were cultivated in TexMACS Research Medium supplemented with 5% human serum type AB and 500 U/mL IL-2. IL-21 (100 ng/mL) was supplemented at day 0 only. For repeated stimulation of the NK cells with B cell derived feeder cells, 100 Gy irradiated EBV-LCL (SMI-EBV-LCL) were added again after several days. Shown in FIG 6 is the adding of EBV-LCL at day 0, 13, 26 and 39 to the NK cells at a ratio of 20:1 whereas the total cell concentration was adjusted to 5.25x10⁵/mL by adding fresh medium containing IL-2. The cells were cultivated at 37°C and 5 % CO₂ for 46 days and at day 7, 9, 11, 18, 20, 23, 32 and/or 33 and 36 the NK cell concentration was diluted to 5x10⁵/mL by adding fresh medium containing IL-2. The NK cell fold expansion was calculated as NK cell number at day of interest divided by NK cell number at start of cultivation. The mean values for NK cell fold expansion of the different donors at the different time points are displayed in FIG 6. Of note, error bars shown in FIG 6 display the range of the different donors and are not for standard deviation. Efficient and constant expansion of the NK cells was achieved over long time and after 46 days 2.7 x10¹¹ mean NK cell fold expansion was reached. In conclusion, adding IL-21 at beginning of the cultivation and repeated stimulation with b cell derived feeder cells enabled the permanent expansion of primary NK cells with outstanding efficacy.

### Example 7: Applying an automated cell processing system for NK cell expansion by use of an optimized method for NK cell expansion

To test the production of NK cells in a clinically applicable way, NK cell expansion was performed in the CliniMACS Prodigy (Miltenyi), a closed system for automated cell processing (WO2009072003A2).

NK cells were cultivated in TexMACS Research Medium supplemented with 5% human serum type AB and 500 U/mL IL-2 together with 100 Gy irradiated EBV-LCL (SMI-EBV-LCL) at a ratio of 1:20. IL-21 (100 ng/mL) was supplemented at day 0 only. The expansion process was performed for three different donors using three different CliniMACS Prodigy^{®} devices. The mean NK cell number at beginning of the cultivation was 1.08x10⁶ NK cells and the NK cells were suspended in 60 mL within the CentriCult Unit of the CliniMACS Prodigy^{®} instrument. The CentriCult Unit was used to culture the cells and a program of the device maintained the cultivation conditions at 37°C and 5% CO₂. The cell concentration was diluted to 3-5x 10⁵ NK cells/mL by adding 60 or 120 mL fresh medium at day 7. Fresh medium was added again at day 9 to the CentriCult Unit until the maximum cultivation volume of 300 mL was reached. 230 mL of the old medium was removed and replaced by 230 mL fresh medium at day 12. At day 14 the cultivation was terminated since the maximum cell concentration that is obtainable by use of the instrument was reached (around 1x10⁷/mL). The NK cell concentration was measured at day 0, 7, 9, 12 and 14 and the total number of NK cells within the CentriCult Unit was calculated. The mean NK cell numbers at the different time points are shown in FIG 7. Error bars in FIG 7 are for standard deviation. A mean number of 3.2x10⁹ NK cells could be produced in two weeks by use of the CliniMACS Prodigy^{®} device.

In conclusion, it is possible to start with a low number of NK cells and produce a therapeutic dose of NK cells within two weeks by use of a cell processing device. Furthermore, based on the results shown in example 6, it would be possible to perform an automated and continuously running NK cell production process with the used instrumentation by repeated harvesting the majority of cells whenever the maximum cell density is reached and re-stimulation of the remaining NK cells with EBV-LCL feeder cells.

### References

Apel M, et al. "Integrated Clinical Scale Manufacturing System for Cellular Products Derived by Magnetic Cell Separation, Centrifugation and Cell Culture" Chemie Ing Tech 2013. 85: 103e10.
Childs RW, Berg R "Bringing natural killer cells to the clinic: ex vivo manipulation." Hematology Am Soc Hematol Educ Program 2013. 2013:234-46
Denman CJ et al., 2012: "Membrane-Bound IL-21 Promotes Sustained Ex Vivo Proliferation of Human Natural Killer Cells", PLoS ONE
Glienke W, et al. "Advantages and applications of CAR-expressing natural killer cells." Front Pharmacol 2015. Feb 12;6:21
Hercend T, et al. "Generation of a cloned NK cell line derived from the «null cell" fraction of human peripheral blood" J Immunol 1982. 129(3):1299-1305
Kim TJ, et al. "Homotypic NK cell-to-cell communication controls cytokine responsiveness of innate immune NK cells." Sci Rep 2014. Dec 5;4:7157
Klingemann H "Are natural killer cells superior CAR drivers?" Oncoimmunology 2014. Apr 15;3:e28147
Leung W, "Infusions of allogeneic natural killer cells as cancer therapy" Clin Cancer Res 2014. Jul 1;20(13):3390-400
Miller JS "Therapeutic applications: natural killer cells in the clinic" Hematology Am Soc Hematol Educ Program 2013. 2013:247-53
Miller JS, et al. "Successful adoptive transfer and in vivo expansion of human haploidenticalNKcells in patients with cancer" Blood 2005. Apr 15;105(8):3051-7
Morris RJ, et al. " A high-efficiency system of natural killer cell cloning." J Immunol Methods 2005. Dec 20;307(1-2):24-33
Oyer JL, et al. "Generation of highly cytotoxic natural killer cells for treatment of acute myelogenous leukemia using a feeder-free, particle-based approach" Biol Blood Marrow Transplant 2015. Apr;21(4):632-9
Rubnitz JE, et al. "NKAML: a pilot study to determine the safety and feasibility of haploidentical natural killer cell trans- plantation in childhood acute myeloid leukemia" J Clin Oncol 2010. 28:955e9.
Sili U, et al. "Production of good manufacturing practice-grade cytotoxic T lymphocytes specific for Epstein-Barr virus, cytomegalovirus and adenovirus to prevent or treat viral infections post-allogeneic hematopoietic stem cell transplant." Cytotherapy 2012 Jan;14(1):7-11

## Claims

1. A method for in-vitro culturing and expanding natural killer (NK) cells in a cell culture medium comprising a population of NK cells, the method comprising
a) adding an effective concentration of interleukin-21 (IL-21) at the beginning of the culturing process to said medium
b) adding repeatedly an effective concentration of interleukin-2 (IL-2) and/or interleukin-15 (IL-15) to said medium
c) adding repeatedly feeder cells or membrane particles thereof to said medium, wherein said feeder cells are B cell derived which are EBV immortalized; and
wherein said expansion of NK cells in said cell culture medium is maintained for at least 3 weeks.

2. The method according to claim 1, wherein said effective concentration of IL-21 in said cell culture medium is between 0.1 and 1000 ng/mL.

3. The method according to claim 1 or 2, wherein said effective concentration of IL-2 in said cell culture system is between 1 U/mL and 5000 U/mL and/or said effective concentration of IL-15 in said cell culture system is between 0.1 and 1000 ng/mL.

4. The method according to any one of claims 1 to 3, wherein said adding repeatedly feeder cells to said medium is performed between day 5 and day 16 after the previous feeder cell addition.

5. The method according to any one of claims 1 to 4, wherein said expansion of NK cells in said cell culture medium is maintained for at least 5 weeks.

6. The method according to any one of claims 1 to 5, wherein said NK cells in a cell culture medium comprising a population of NK cells are purified NK cells.

7. The method of any one of claims 1 to 6, wherein the starting concentration of said NK cells in a cell culture medium comprising a population of NK cells is between 20 cells/ mL and 2x10⁷ cells/mL.

8. The method of any one of claims 1 to 7, wherein the starting concentration of said NK cells in a cell culture medium comprising a population of NK cells is between 20 cells/ mL and 2.5x10⁴ cells/mL.

9. The method of any one of claims 1 to 8, wherein said feeder cells are genetically non-modified cells with exception of the integration of the EBV genome.

10. The method of any one of claims 1 to 9, wherein said NK cells in a cell culture medium comprising a population of NK cells are genetically modified.

11. The method according to claim 10, wherein said genetically modified NK cells express a chimeric antigen receptor (CAR).

12. The method according to any one of claims 1 to 11, wherein said method is performed in an automated process.

13. The method according to claim 12, wherein said process is performed in a closed system.

14. A pharmaceutical composition comprising a population of NK cells produced according to the method of claims 1-13.
